# EUROPÄISCHE PATENTSCHRIFT

(11) **EP 0 941 742 B1**
(45) Veröffentlichungstag und Bekanntmachung des Hinweises auf die Patenterteilung: **30.07.2003**
(21) Anmeldenummer: 98123541.9
(22) Anmeldetag: 09.12.1998
(51) Int. Cl.: A61M 5/32, A61M 5/34

(54) **Verfahren zur berührungslosen Überwachung auf vorgeschriebenen Sitz einer auf die Kanüle einer medizinischen Spritze aufgesetzten Nadelschutzkappe und Vorrichtung zur Durchführung des Verfahrens**
Method for the contactless monitoring of the required position of a protection cup on a medical syringe and apparatus for carrying out the method
Procédé pour le contrôle sans contact de la position prescrite du capuchon de protection de l'aiguille sur la canule d'une seringue médicale ainsi que l'appareil pour la mise en oeuvre de ce procédé

(30) Priorität: 19.02.1998 DE 19806971
(43) Veröffentlichungstag der Anmeldung: 15.09.1999
(73) Patentinhaber: Arzneimittel GmbH Apotheker Vetter & Co. Ravensburg, D-88212 Ravensburg (DE)
(72) Erfinder: Vetter, Helmut, 88212 Ravensburg (DE); Mossig, Stefan, 88214 Ravensburg (DE); Vetter, Udo J., 88214 Ravensburg (DE)
(74) Vertreter: Dziewior, Joachim, Dipl.-Phys. Dr.

(56) Entgegenhaltungen:
- EP-A- 0 410 734
- EP-A- 0 769 708
- DE-A- 3 215 289
- US-A- 3 623 210
- US-A- 5 375 596

## Beschreibung

Die Erfindung betrifft ein Verfahren zur berührungslosen Überwachung auf vorgeschriebenen Sitz einer auf die Kanüle einer medizinischen Spritze aufgesetzten Nadelschutzkappe, insbesondere zur Prüfung auf beim Aufsetzen der Nadelschutzkappe radial ausweichende und dadurch die Nadelschutzkappe durchstechende Kanülenspitzen. Weiter betrifft die Erfindung eine Vorrichtung zur Durchführung des Verfahrens.

Beim Aufsetzen von Nadelschutzkappen im Herstellungsprozeß von insbesondere vorgefüllten Spritzen ist zu beobachten, daß teilweise die Kanüle nicht genau konzentrisch in die Nadelschutzkappe einsticht, sondern radial ausweicht. Dies kann beispielsweise durch geringfügige Inhomogenitäten in der Nadelschutzkappe oder durch nicht exakt zentrische Führung beim Aufsetzen verursacht werden. Je nach Grad des Ausweichens der Kanüle kann diese nach außen aus der Nadelschutzkappe austreten, so daß nach anschließender Befüllung des Spritzenzylinders dessen Inhalt über die Kanüle Verbindung zur umgebenden Atmosphäre hätte und somit Keime in das pharmazeutische Produkt eindringen könnten.

Um solche "defekten" Spritzenkörper aus dem Produktionsablauf zu nehmen, erfolgt bisher eine visuelle Kontrolle, durch die - deutlich sichtbar - durchstoßende Nadelschutzkappen erkannt und sodann die Spritzenkörper entfernt werden können (siehe zum Beispiel DE-A-3 215 289).

Schwieriger oder auch nicht erkennbar sind jene Fälle, bei denen die Kanülenspitze gerade die Außenmantelfläche der Kanülenschutzkappe erreicht oder sich nach erfolgtem Durchstich soweit zurückzieht, daß sie nicht mehr nach außen vorsteht. Auch in letzterem Fall kann eine Kontamination der Kanüle nicht ausgeschlossen werden, da Keime über den eingestochenen Kanal eindringen können.

Schließlich ist wünschenswert, auch solche Spritzenkörper zu erkennen und auszusortieren, bei denen der Abstand der Kanülenspitze von der Außenmantelfläche der Kanülenschutzkappe einen Mindestabstand unterschreitet, der bei etwa 0,1 bis 0,2 mm liegen kann.

Hiervon ausgehend liegt der Erfindung die Aufgabe zugrunde, ein Verfahren und eine Vorrichtung anzugeben, durch das bzw. die eine zuverlässige Prüfung und anschließende Aussonderung solcher Spritzenkörper gewährleistet ist, bei denen die auf die Kanüle aufgesetzte Kanülenschutzkappe von der Kanülenspitze durchstochen wurde oder die Kanülenspitze einen Mindestabstand von Außenmantelfläche der Kanülenschutzkappe unterschreitet.

Diese Aufgabe wird in verfahrensmäßiger Hinsicht dadurch gelöst, daß an die Kanüle einerseits und an eine die Nadelschutzkappe umgebende Gegenelektrode andererseits eine Hochspannung angelegt wird, daß sodann der in einer der elektrischen Zuleitungen zur Kanüle bzw. zur Gegenelektrode fließende Strom überwacht wird und daß bei Überschreitung eines vorgegebenen Stromwertes die der Prüfung unterzogene Spritze als fehlerhaft gekennzeichnet und aus dem weiteren Fertigungsprozeß ausgeschieden wird, wobei die Nadelschutzkappe aus einem Material von gegenüber dem umgebenden Gas höherer Dielektrizitätskonstante besteht.

Der durch die Erfindung erreichte Vorteil besteht zunächst darin, daß die Prüfung nicht mehr visuell, sondern automatisiert erfolgen kann, wodurch nicht nur ein höherer Durchsatz, sondern auch eine zuverlässigere Überwachung gewährleistet wird. Darüber hinaus werden auch solche - visuell kaum oder nicht erkennbare - Spritzenkörper entdeckt und können ausgeschieden werden, bei denen ein Durchstich erfolgt, die Kanülenspitze jedoch wieder ins Innere der Nadelschutzkappe zurückgetreten ist. Schließlich erfaßt das erfindungsgemäße Verfahren auch Spritzenkörper, die an sich visuell keineswegs zu beanstanden wären, bei denen aber wegen des Unterschreitens eines Mindestabstands zwischen der Kanülenspitze und der Außenmantelfläche der Kanülenschutzkappe zumindest ein Kontaminationsrisiko besteht.

In bevorzugter Ausgestaltung der Erfindung ist vorgesehen, daß die Zuleitung zur Kanüle über einen Prüfdorn erfolgt, der zur Messung in den Spritzenzylinder eingeführt wird. Das Verfahren läßt sich grundsätzlich bei jenen Spritzenkörpern anwenden, bei denen die Kanüle sich bis zum bzw. bis in den Spritzenzylinder erstreckt. Die Kanüle ist hierbei häufig in den Glasspritzenkörper eingeklebt. Dabei kann der Prüfdorn zur Messung entweder in direkten Kontakt mit oder zumindest in geringen Abstand zu dem in den Spritzenzylinder mündenden Ende der Kanüle gebracht werden, da infolge der angewandten Hochspannung eine unmittelbare Kontaktgabe nicht erforderlich ist.

Zugleich besteht dabei die Möglichkeit, daß über den als Hohlnadel ausgebildeten Prüfdorn vor und/oder nach der Prüfung eine Begasung des Spritzenzylinders erfolgt.

Um eine weitere Steigerung des Durchsatzes zu erreichen, sieht die Erfindung vor, daß die Prüfung der Spritzen an mehreren, vorzugsweise vier oder sechs Prüfstationen gleichzeitig durch an jeder Prüfstation vorgesehene, eigene Hochspannungsversorgungen und Strommeßeinrichtungen erfolgt.

Da die Spritzen üblicherweise hängend in die Prüfstation überführt werden, empfielt es sich, daß die Spritzen zur Durchführung der Messung mit den Nadelschutzkappen in die als U-Profil ausgebildete Gegenelektrode von deren Stirnseite her eingebracht werden.

Als günstig hat es sich herausgestellt, wenn als Prüfspannung eine Gleichspannung in der Größenordnung von 10 KV zur Anwendung kommt.

Da die Spritzenkörper im Anschluß an die Prüfung auf Grund des hohen elektrischen Feldes eine elektrostatische Aufladung aufweisen, besteht bei der sich anschließenden Befüllung die Gefahr, daß ein an der Befüllnadel hängender Flüssigkeitstropfen unter dem Einfluß des Feldes "zerstäubt" und dadurch eine - unerwünschte - Benetzung der Innenwand des Spritzenkörpers in einem Bereich erfolgt, in den später der Stopfen eingesetzt wird. Das sich so in dieser Zone absetzende pharmazeutische Produkt kann eine Unterwanderung der Lamellen des Stopfens durch Keime begünstigen. Um dies zu vermeiden, ist vorgesehen, daß die Spritzen im Anschluß an die Prüfstation eine Entladestation zur Beseitigung der elektrostatischen Aufladung durchlaufen.

In vorrichtungsmäßiger Hinsicht wird die oben genannte Aufgabe durch eine Vorrichtung mit einer oder mehreren Prüfstationen für die bereits mit einer Nadelschutzkappe versehenen medizinischen Spritzen gelöst, bei der jede Prüfstation für die zu prüfende Spritze einen im wesentlichen konzentrisch zur Spritze ausgerichteten, als Elektrode ausgebildeten Prüfdorn aufweist, der in axialer Richtung ins Innere der Spritze verstellbar ist, wobei ferner eine der bzw. den Spritzenkanülen zugeordnete Gegenelektrode sowie ein Hochspannungsgenerator vorgesehen ist, wobei über eine Steuer- und Meßeinrichtung der Prüfdorn und die Gegenelektrode mit der Spannung des Hochspannungsgenerators beaufschlagbar ist und der sich einstellende Stromfluß von der Meßeinrichtung gemessen und erfaßt wird.

Zweckmäßigerweise ist dabei der Prüfdorn als Hohlnadel zur Begasung des Spritzenzylinders vor und/oder nach der Prüfung ausgebildet.

Weiter ist es hierbei im Rahmen der Erfindung von Vorteil, wenn der Prüfdorn mit einer lediglich die Spitze freilassenden Isolierung versehen ist.

Da die - meist taktweise - Bearbeitung der Spritzenkörper zu mehreren erfolgt, empfielt es sich, daß vorzugsweise vier oder sechs Prüfstationen vorgesehen sind, wobei zur gleichzeitigen Messung an jeder Prüfstation diese jeweils mit einer eigenen Hochspannungsversorgung und Strommeßeinrichtung versehen sind.

In besonders einfacher und daher im Rahmen der Erfindung bevorzugter Ausgestaltung ist die Gegenelektrode als U-Profil ausgebildet. Es sind jedoch - insbesondere bei speziellen Anwendungsfällen - auch andere Gestaltungen denkbar. So kann die Gegenelektrode beispielsweise auch als die Nadelschutzkappe umgebende Kugelkalotte ausgebildet sein.

Der Hochspannungsgenerator ist vorteilhafterweise zur Erzeugung einer Gleichspannung in der Größenordnung von 10 KV, vorzugsweise 12 bis 14 KV eingerichtet.

Um eine Entladung der Spritzenkörper nach der Prüfung vornehmen zu können, ist der bzw. den Prüfstationen eine Entladestation zur Beseitigung der elektrostatischen Aufladung der Spritzen nachgeschaltet, die eine ionisierte Entladungswolke erzeugt, die von den Spritzen durchlaufen wird.

Als vorteilhaft hat es sich erwiesen, wenn die Entladestation von einem mit mehreren Elektrodenspitzen versehenen Ionisierkopf gebildet ist, wobei die Elektrodenspitzen von einer Wechselhochspannung von etwa 5 KV gespeist werden. Dabei ist es weiter von Vorteil, wenn die Elektrodenspitzen kreisförmig zueinander angeordnet sind. Es besteht jedoch auch die Möglichkeit, daß der Ionisierkopf leistenförmig in rechteckiger, quadratischer oder ovaler Gestalt ausgebildet ist. Besonders vorteilhaft kann es sein, wenn die Elektrodenspitzen in ihrer Anordnung der Gestalt des Spritzenkörpers und gegebenenfalls der den Spritzenkörper tragenden Halteeinheit angepaßt sind.

Schließlich können zwischen den Elektrodenspitzen Ausblasöffnungen vorgesehen sein, die von einer gemeinsamen Gaszufuhr gespeist werden. Dadurch kann durch Einblasen von Gas, beispielsweise Luft, bedarfsweise eine Beeinflussung der Entladungswolke erreicht werden.

Im folgenden wird die Erfindung an einem in der Zeichnung dargestellten Ausführungsbeispiel näher erläutert; es zeigen:
- Fig. 1: eine Prüfstation in nur schematischer Darstellung,
- Fig. 2: die der Prüfstation nachgeordnete Entladestation,
- Fig. 3: eine alternative Gestaltung der Entladestation.

Die in der Zeichnung nur schematisch dargestellte Vorrichtung besteht aus in der Regel mehreren Prüfstationen 1 für die bereits mit einer Nadelschutzkappe 2 versehenen medizinischen Spritzen 3, die im Anschluß an einen hier nicht weiter zu erörternden Reinigungsprozeß mit einem pharmazeutischen Produkt befüllt und danach - steril - verpackt werden.

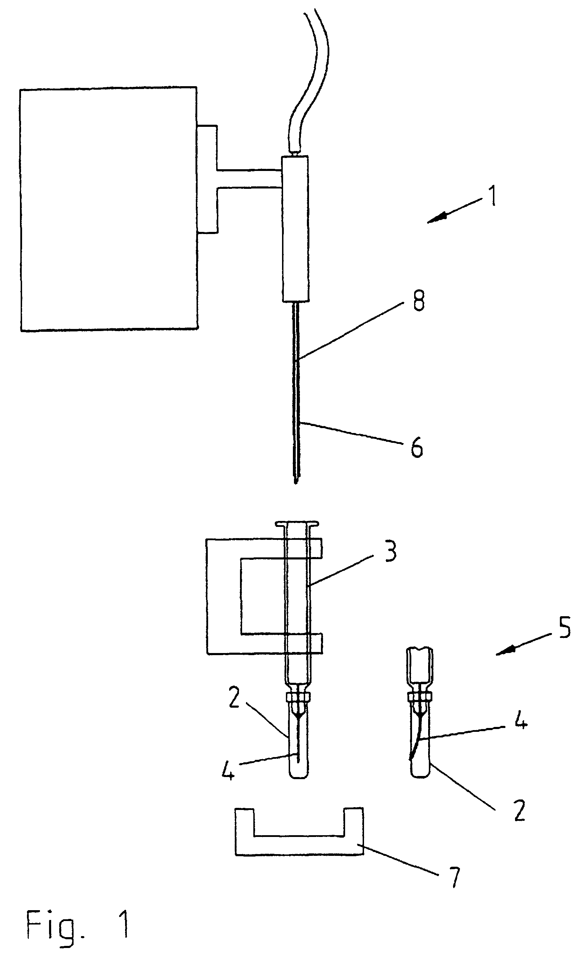

Beim Aufsetzen von Nadelschutzkappen 2 auf Spritzenkörper 3 ist zu beobachten, daß die Kanüle 4 bisweilen nicht genau konzentrisch in die Nadelschutzkappe 2 eindringt, sondern ihre Spitze mehr oder weniger radial ausweicht, wie dies in Fig. 1 rechts neben der Spritze 3 durch die Darstellung 5 angedeutet ist. Ursache hierfür können geringfügige Inhomogenitäten in der Nadelschutzkappe 2 oder eine nicht exakt zentrische Führung beim Aufsetzen der Nadelschutzkappe 2 sein. Somit kann die Kanülenspitze nach außen aus der Nadelschutzkappe 2 austreten, so daß nach anschließender Befüllung des Spritzenzylinders dessen Inhalt über die Kanüle 4 mit der umgebenden Atmosphäre in Verbindung stünde. Damit könnten Keime in das pharmazeutische Produkt eindringen.

Es besteht aber auch die Gefahr, daß die Kanülenspitze die Außenmantelfläche der Kanülenschutzkappe 2 nur eben erreicht oder sich nach erfolgtem Durchstich wieder soweit zurückzieht, daß sie nicht mehr nach außen vorsteht. Auch dann kann eine Kontamination der Kanüle 4 nicht ausgeschlossen werden, da Keime über den in die Kanülenschutzkappe 2 eingestochenen Kanal eindringen können.

Schließlich sollen auch solche Spritzenkörper 3 aus dem Produktionsprozeß genommen werden, bei denen der Abstand der Kanülenspitze von der Außenmantelfläche der Kanülenschutzkappe 2 einen Mindestabstand unterschreitet, der etwa bei 0,1 bis 0,2 mm liegt.

Um solche Spritzenkörper 3 zuverlässig zu ermitteln, weist die Prüfstation 1 für die zu prüfende Spritze 3 einen im wesentlichen konzentrisch zur Spritze 3 ausgerichteten, als Elektrode ausgebildeten Prüfdorn 6 auf, der in axialer Richtung ins Innere der Spritze 3 verstellbar ist. Ferner ist eine der bzw. den Spritzenkanülen 4 zugeordnete Gegenelektrode 7 sowie ein in der Zeichnung nicht dargestellter Hochspannungsgenerator vorgesehen, wobei über eine Steuer- und Meßeinrichtung der Prüfdorn 6 und die Gegenelektrode 7 mit der Spannung des Hochspannungsgenerators beaufschlagbar ist.

Ist eine Nadelschutzkappe 2 durchstochen bzw. der Abstand zwischen Kanülenspitze und äußerer Mantelfläche der Nadelschutzkappe 2 zu gering, so kommt es nach Anlegen der Hochspannung zu einer Elektronenemmision und somit zu einem Stromfluß zwischen der Kanülenspitze und der Gegenelektrode 7. Wird also nach der Spannungsbeaufschlagung der sich einstellende Stromfluß gemessen und erfaßt, so lassen sich in obigem Sinn "defekte" Spritzenkörper 3 ermitteln und aus dem Produktionsablauf entfernen.

Damit ermöglicht diese Vorrichtung eine berührungslose Überwachung auf vorgeschriebenen Sitz der auf die Kanüle 4 der Spritze 3 aufgesetzten Nadelschutzkappe 2, indem Bei Überschreitung eines vorgegebenen Stromwertes die der Prüfung unterzogene Spritze als fehlerhaft gekennzeichnet und aus dem weiteren Fertigungsprozeß ausgeschieden wird. Dabei muß die Nadelschutzkappe 2 aus einem Material von gegenüber dem umgebenden Gas - üblicherweise Luft - höherer Dielektrizitätskonstante bestehen.

Die Zuleitung zur Kanüle 4 erfolgt über den Prüfdorn 6, der zur Messung in den Spritzenzylinder 3 eingeführt wird. Dabei kann der Prüfdorn 6 zur Messung entweder in direkten Kontakt mit dem in den Spritzenzylinder mündenden Ende der Kanüle 4 oder aber zumindest in geringen Abstand hierzu gebracht werden, was insofern ausreichend ist, als die Spannung - bei beschädigter Kanülenschußkappe 2 - vom Prüfdorn 6 zur Kanüle 4 überschlagen kann.

Der Prüfdorn 6 ist als Hohlnadel ausgebildet, wodurch die Möglichkeit besteht, ohne zusätzlichen Arbeitsschritt vor und/oder nach der Prüfung eine Begasung des Spritzenzylinders vorzunehmen.

Der Prüfdorn 6 ist mit einer lediglich die Spitze freilassenden Isolierung 8 versehen, wodurch einerseits ein Berührungschutz gegeben und die Gefahr unkontollierbarer Überschläge ausgeschlossen ist.

Für die taktweise Bearbeitung der Spritzenkörper 3 sind vorzugsweise vier oder sechs Prüfstationen 1 vorgesehen, wobei zur gleichzeitigen Messung an jeder Prüfstation 1 diese jeweils mit einer eigenen Hochspannungsversorgung und Strommeßeinrichtung versehen sind.

Die Gegenelektrode 7 ist einfach als U-Profil ausgebildet, in das die Spritzenkörper 3 mit den Kanülenschutzkappen 2 von der Stirnseite her zur Prüfung einlaufen, sofern sie - entgegen der Darstellung in der Fig. 1 - in das Profil eintauchen. Es hat sich gezeigt, daß die Form der Gegenelektrode 7 wenig Einfluß auf die Prüfgenauigkeit hat. Ebenso sind Klimaeinflüsse wie Luftdruck, Luftfeuchte und Temperatur von untergeordneter Bedeutung.

Der Hochspannungsgenerator ist zur Erzeugung einer Gleichspannung in der Größenordnung von 10 KV, vorzugsweise 12 bis 14 KV eingerichtet.

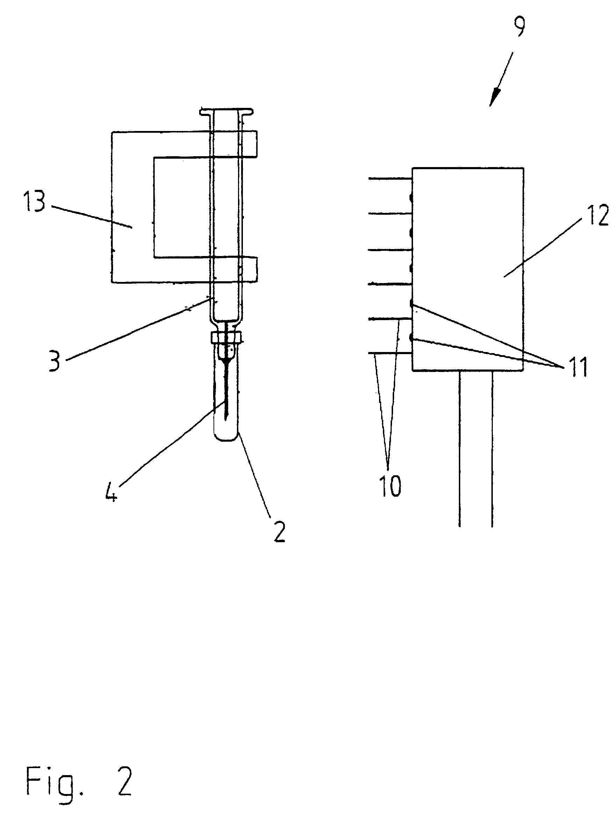

Um die Spritzenkörper 3 im Anschluß an die Prüfung von ihrer elektrostatischen Aufladung zu befreien, ist eine Entladestation 9 gemäß Fig. 2 vorgesehen. Erfolgt die Entladung nicht, so besteht bei der anschließenden Befüllung des Spritzenkörpers 3 die Gefahr, daß ein an der Befüllnadel hängender Flüssigkeitstropfen sich löst und unter dem Einfluß des elektrischen Feldes "zerstäubt" wird. Dadurch kann eine - unerwünschte - Benetzung der Innenwand des Bereiches des Spritzenkörpers 3 erfolgen, in den später der Stopfen eingesetzt wird. Das sich auf diese Weise in dieser Zone absetzende pharmazeutische Produkt kann eine Unterwanderung der Lamellen des Stopfens durch Keime begünstigen. Diese Gefahr wird vermieden, wenn die Spritzen 3 im Anschluß an die Prüfstation 1 die Entladestation 9 zur Beseitigung der elektrostatischen Aufladung durchlaufen.

Im einzelnen erzeugt die Entladestation 9 eine in der Fig. 2 durch Punkte angedeutete ionisierte Entladungswolke, die die Spritzen 3 unter Abgabe ihrer elektrostatischen Ladung durchlaufen.

Dazu ist die Entladestation 9 von einem mit mehreren Elektrodenspitzen 10 versehenen Ionisierkopf 12 gebildet, wobei die Elektrodenspitzen 10 von einer Wechselhochspannung von etwa 5 KV gespeist werden.

Die Elektrodenspitzen 10 können, wie aus der Zeichnung zu erkennen, kreisförmig zueinander angeordnet sein.

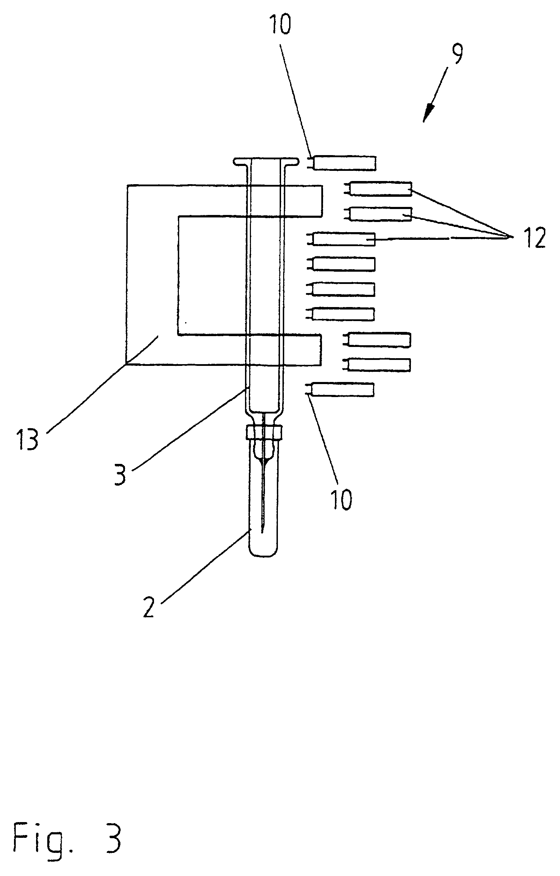

Es sind jedoch auch beliebige andere Anordnungen denkbar, insbesondere können die Elektrodenspitzen 10 in ihrer Anordnung der Gestalt des Spritzenkörpers 3 und gegebenenfalls der den Spritzenkörper tragenden Halteeinheit 13 angepaßt sein, wie dies in Fig. 3 angedeutet ist.

Zwischen den Elektrodenspitzen 10 sind Ausblasöffnungen 11 vorgesehen, die von einer gemeinsamen Gaszufuhr gespeist werden können, wodurch Einfluß auf die Form der Entladungswolke und somit den Entladungsvorgang genommen werden kann.

## Patentansprüche

1. Verfahren zur berührungslosen Überwachung auf vorgeschriebenen Sitz einer auf die Kanüle (4) einer medizinischen Spritze (3) aufgesetzten Nadelschutzkappe (2), insbesondere zur Prüfung auf beim Aufsetzen der Nadelschutzkappe (4) radial ausweichende und dadurch die Nadelschutzkappe (4) durchstechende Kanülenspitzen, **dadurch gekennzeichnet, daß** an die Kanüle (4) einerseits und an eine die Nadelschutzkappe (2) umgebende Gegenelektrode (7) andererseits eine Hochspannung angelegt wird, daß sodann der in einer der elektrischen Zuleitungen zur Kanüle (4) bzw. zur Gegenelektrode (7) fließende Strom überwacht wird und daß bei Überschreitung eines vorgegebenen Stromwertes die der Prüfung unterzogene Spritze (3) als fehlerhaft gekennzeichnet und aus dem weiteren Fertigungsprozeß ausgeschieden wird, wobei die Nadelschutzkappe (2) aus einem Material von gegenüber dem umgebenden Gas höherer Dielektrizitätskonstante besteht.

2. Verfahren nach Anspruch 1, **dadurch gekennzeichnet, daß** die Zuleitung zur Kanüle (4) über einen Prüfdorn (6) erfolgt, der zur Messung in den Spritzenzylinder (3) eingeführt wird.

3. Verfahren nach einem der Ansprüche 1 bis 3, **dadurch gekennzeichnet, daß** der Prüfdorn (6) zur Messung in direkten Kontakt mit oder zumindest in geringen Abstand zu dem in den Spritzenzylinder mündenden Ende der Kanüle (4) gebracht wird.

4. Verfahren nach Anspruch 2, **dadurch gekennzeichnet, daß** über den als Hohlnadel ausgebildeten Prüfdorn (6) vor und/oder nach der Prüfung eine Begasung des Spritzenzylinders erfolgt.

5. Verfahren nach den Ansprüchen 1 bis 4, **dadurch gekennzeichnet, daß** die Prüfung der Spritzen (3) an mehreren, vorzugsweise vier oder sechs Prüfstationen (1) gleichzeitig durch an jeder Prüfstation (1) vorgesehene, eigene Hochspannungsversorgungen und Strommeßeinrichtungen erfolgt.

6. Verfahren nach den Ansprüchen 1 bis 5, **dadurch gekennzeichnet, daß** die Spritzen (3) zur Durchführung der Messung mit den Nadelschutzkappen (2) in die als U-Profil ausgebildete Gegenelektrode (7) von deren Stirnseite her eingebracht werden.

7. Verfahren nach den Ansprüchen 1 bis 6, **dadurch gekennzeichnet, daß** als Prüfspannung eine Gleichspannung in der Größenordnung von 10 KV zur Anwendung kommt.

8. Verfahren nach den Ansprüchen 1 bis 7, **dadurch gekennzeichnet, daß** die Spritzen (3) im Anschluß an die Prüfstation (1) eine Entladestation (9) zur Beseitigung der elektrostatischen Aufladung durchlaufen.

9. Vorrichtung zur Durchführung des Verfahrens nach den Ansprüchen 1 bis 8, mit einer oder mehreren Prüfstationen (1) für die bereits mit einer Nadelschutzkappe (2) versehenen medizinischen Spritzen (3), **dadurch gekennzeichnet, daß** jede Prüfstation (1) für die zu prüfende Spritze (3) einen im wesentlichen konzentrisch zur Spritze (3) ausgerichteten, als Elektrode ausgebildeten Prüfdorn (6) aufweist, der in axialer Richtung ins Innere der Spritze (3) verstellbar ist, daß ferner eine der bzw. den Spritzenkanülen (4) zugeordnete Gegenelektrode (7) sowie ein Hochspannungsgenerator vorgesehen ist, wobei über eine Steuer- und Meßeinrichtung der Prüfdorn (6) und die Gegenelektrode (7) mit der Spannung des Hochspannungsgenerators beaufschlagbar ist und der sich einstellende Stromfluß von der Meßeinrichtung gemessen und erfaßt wird.

10. Vorrichtung nach Anspruch 9, **dadurch gekennzeichnet, daß** der Prüfdorn (6) als Hohlnadel zur Begasung des Spritzenzylinders vor und/oder nach der Prüfung ausgebildet ist.

11. Vorrichtung nach Anspruch 9 oder 10, **dadurch gekennzeichnet, daß** der Prüfdorn (6) mit einer lediglich die Spitze freilassenden Isolierung (8) versehen ist.

12. Vorrichtung nach den Ansprüchen 9 bis 11, **dadurch gekennzeichnet, daß** vorzugsweise vier oder sechs Prüfstationen (1) vorgesehen sind, wobei zur gleichzeitigen Messung an jeder Prüfstation (1) diese jeweils mit einer eigenen Hochspannungsversorgung und Strommeßeinrichtung versehen sind.

13. Vorrichtung nach den Ansprüchen 9 bis 12, **dadurch gekennzeichnet, daß** die Gegenelektrode (7) als U-Profil ausgebildet ist.

14. Vorrichtung nach den Ansprüchen 9 bis 12, **dadurch gekennzeichnet, daß** die Gegenelektrode (7) als die Nadelschutzkappe (2) umgebende Kugelkalotte ausgebildet ist.

15. Vorrichtung nach den Ansprüchen 9 bis 14, **dadurch gekennzeichnet, daß** der Hochspannungsgenerator zur Erzeugung einer Gleichspannung in der Größenordnung von 10 KV, vorzugsweise 12 bis 14 KV eingerichtet ist.

16. Vorrichtung nach den Ansprüchen 9 bis 15, **dadurch gekennzeichnet, daß** der bzw. den Prüfstationen (1) eine Entladestation (9) zur Beseitigung der elektrostatischen Aufladung der Spritzen (3) nachgeschaltet ist, die eine ionisierte Entladungswolke erzeugt, die von den Spritzen (3) durchlaufen wird.

17. Vorrichtung nach Anspruch 16, **dadurch gekennzeichnet, daß** die Entladestation (9) von einem mit mehreren Elektrodenspitzen (10) versehenen Ionisierkopf (12) gebildet ist, wobei die Elektrodenspitzen von einer Wechselhochspannung von etwa 5 KV gespeist werden.

18. Vorrichtung nach Anspruch 17, **dadurch gekennzeichnet, daß** die Elektrodenspitzen (10) kreisförmig zueinander angeordnet sind.

19. Vorrichtung nach Anspruch 17, **dadurch gekennzeichnet, daß** der Ionisierkopf (12) leistenförmig in rechteckiger, quadratischer oder ovaler Gestalt ausgebildet ist.

20. Vorrichtung nach Anspruch 17, 18 oder 19, **dadurch gekennzeichnet, daß** die Elektrodenspitzen (10) in ihrer Anordnung der Gestalt des Spritzenkörpers (3) und gegebenenfalls der den Spritzenkörper tragenden Halteeinheit (13) angepaßt sind.

21. Vorrichtung nach enem der Ansprüche 17 bis 20, **dadurch gekennzeichnet, daß** zwischen den Elektrodenspitzen (10) Ausblasöffnungen (11) vorgesehen sind, die von einer gemeinsamen Gaszufuhr gespeist werden.

## Claims

1. A method of contactlessly monitoring for the prescribed fit of a needle protective cap (2) fitted on to the hollow needle (4) of a medical syringe (3), in particular for checking for hollow needle tips which when the needle protective cap (2) is fitted deflect radially and thereby pierce the needle protective cap (2), **characterised in that** a high voltage is applied to the hollow needle (4) on the one hand and on the other hand to a counterpart electrode (7) surrounding the needle protective cap (2), that then the current flowing in one of the electrical feed lines to the hollow needle (4) or to the counterpart electrode (7) respectively is monitored and that when a predetermined current value is exceeded the syringe (3) subjected to the checking procedure is identified as defective and removed from the further manufacturing procedure, wherein the needle protective cap (2) comprises a material of a dielectric constant which is higher than the surrounding gas.

2. A method according to claim 1 **characterised in that** the feed to the hollow needle (4) is effected by way of a checking bar (6) which for the measurement procedure is introduced into the syringe cylinder (3).

3. A method according to one of claims 1 and 2 **characterised in that** the checking bar (6) for the measurement procedure is brought into direct contact with or at least at a small spacing relative to the end of the hollow needle (4) which opens into the syringe cylinder.

4. A method according to claim 2 **characterised in that** the syringe cylinder is supplied with gas by way of the checking bar (6) which is in the form of a hollow needle, prior to and/or after the checking operation.

5. A method according to claims 1 to 4 **characterised in that** the operation of checking the syringes (3) is effected at a plurality of and preferably four or six checking stations (1) simultaneously by particular high-voltage supplies and current measuring devices provided at each checking station (1).

6. A method according to claims 1 to 5 **characterised in that** for carrying out the measuring operation the syringes (3) are introduced with the needle protective caps (2) into the counterpart electrode (7) which is in the form of a U-shaped member, from the end thereof.

7. A method according to claims 1 to 6 **characterised in that** the checking voltage used is a dc voltage of the order of magnitude of 10 KV.

8. A method according to claims 1 to 7 **characterised in that** subsequently to the checking station (1) the syringes pass through a discharging station (9) for eliminating the electrostatic charge.

9. Apparatus for carrying out the method according to claims 1 to 8 comprising one or more checking stations (1) for the medical syringes (3) which are already provided with a needle protective cap (2), **characterised in that** each checking station (1) for the syringe (3) to be checked has a checking bar (6) which is oriented substantially concentrically with respect to the syringe (3) and which is in the form of an electrode and which is displaceable in the axial direction into the interior of the syringe (3), that in addition there are provided a counterpart electrode (7) associated with the syringe hollow needle or needles (4) and a high-voltage generator, wherein the checking bar (6) and the counterpart electrode (7) can be supplied with the voltage of the high-voltage generator by way of a control and measuring device and the current flow which is produced is measured and detected by the measuring device.

10. Apparatus according to claim 9 **characterised in that** the checking bar (6) is in the form of a hollow needle for supplying gas to the syringe cylinder prior to and/or after the checking operation.

11. Apparatus according to claim 9 or claim 10 **characterised in that** the checking bar (6) is provided with an insulation (8) which exposes only the tip.

12. Apparatus according to claims 9 to 11 **characterised in that** there are preferably four or six checking stations (1), wherein for simultaneous measurement at each checking station (1) they are each provided with their own high-voltage supply and current measuring device.

13. Apparatus according to claims 9 to 12 **characterised in that** the counterpart electrode (7) is in the form of a U-shaped member.

14. Apparatus according to claims 9 to 12 **characterised in that** the counterpart electrode (7) is in the form of a portion of a sphere surrounding the needle protective cap (2).

15. Apparatus according to claims 9 to 14 **characterised in that** the high-voltage generator is adapted to produce a dc voltage of the order of magnitude of 10 KV, preferably 12 to 14 KV.

16. Apparatus according to claims 9 to 15 **characterised in that** connected downstream of the checking station or stations (1) is a discharging station (9) for eliminating the electrostatic charge of the syringes (3), which produces an ionised discharge cloud through which the syringes (3) pass.

17. Apparatus according to claim 16 **characterised in that** the discharging station (9) is formed by an ionisation head (12) provided with a plurality of electrode points (10), wherein the electrode points are fed by a high ac voltage of about 5 KV.

18. Apparatus according to claim 17 **characterised in that** the electrode points (10) are arranged in a circular array relative to each other.

19. Apparatus according to claim 17 **characterised in that** the ionisation head (12) is of a strip-shaped form of a rectangular, square or oval configuration.

20. Apparatus according to claims 17, 18 or 19 **characterised in that** the electrode points (10) are adapted in their arrangement to the configuration of the syringe body (3) and possibly the holding unit (13) carrying the syringe body.

21. Apparatus according to one of claims 17 to 20 **characterised in that** provided between the electrode points (10) are blowing-out openings (11) which are fed by a common gas supply.

## Revendications

1. Procédé pour le contrôle sans contact du positionnement correct du capuchon de protection d'aiguille (2) sur la canule (4) d'une seringue (3) médicale, en particulier pour détecter les extrémités de canule qui s'écartent radialement lors de la mise en place du capuchon d'aiguille (4) et percent ainsi ledit capuchon d'aiguille (2), **caractérisé en ce qu'**on applique une haute tension à la canule (4) d'une part et à une contre-électrode (7) entourant le capuchon de protection d'aiguille (2) d'autre part, **en ce qu'**ensuite on surveille le courant qui circule dans l'une des lignes menant à la canule (4) ou à la contre-électrode (7) et **en ce qu'**en cas de dépassement d'une valeur de courant prédéfinie, on marque comme défectueuse la seringue (7) contrôlée et on l'élimine de la suite du cycle de fabrication, le capuchon de protection d'aiguille (2) étant réalisé en un matériau qui présente une constante diélectrique supérieure à celle du gaz environnant.

2. Procédé selon la revendication 1, **caractérisé en ce que** la liaison avec la canule (4) est réalisée par l'intermédiaire d'une broche de contrôle (6), qui est introduite dans le cylindre de seringue (3) pour la mesure.

3. Procédé selon une des revendications 1 à 3, **caractérisé en ce que** la broche de contrôle (6), pour la mesure, est amenée en contact direct avec ou au moins à une faible distance de l'extrémité de la canule (4) débouchant dans le cylindre de seringue.

4. Procédé selon la revendication 2, **caractérisé en ce qu'**un lavage au gaz du cylindre de seringue est réalisé avant et/ou après le contrôle, à travers la broche de contrôle (6) configurée en aiguille creuse.

5. Procédé selon une des revendications 1 à 4, **caractérisé en ce que** le contrôle des seringues (3) a lieu simultanément dans plusieurs postes de contrôle (1), de préférence quatre ou six, à l'aide d'alimentations à haute tension et de dispositifs de mesure de courant propres, prévus dans chaque poste de contrôle (1).

6. Procédé selon une des revendications 1 à 5, **caractérisé en ce que** les seringues (3), pour effectuer la mesure avec les capuchons de protection d'aiguille (2), sont placées dans la contre-électrode (7) réalisée conformée en profilé en U, à partir de la face frontale de celui-ci.

7. Procédé selon une des revendications 1 à 6, **caractérisé en ce qu'**on utilise comme tension de contrôle une tension continue de l'ordre de 10 kV.

8. Procédé selon une des revendications 1 à 7, **caractérisé en ce que** les seringues (3), après le poste de contrôle (9) traversent un poste de décharge (9) pour éliminer la charge électrostatique.

9. Dispositif pour la mise en oeuvre du procédé selon les revendications 1 à 8, avec un ou plusieurs postes de contrôle (1) pour les seringues (3) médicales prémunies d'un capuchon de protection d'aiguille (2), **caractérisé en ce que** chaque poste de contrôle (1) comporte, pour la seringue (3) à contrôler, une broche de contrôle (6) essentiellement concentrique avec la seringue (3), conformée en électrode, qui peut être déplacée dans la direction axiale à l'intérieur de la seringue (3), **en ce qu'**il est prévu en outre une contre-électrode (7) associée à la ou aux canule(s) de seringue (4) ainsi qu'un générateur de haute tension, la broche de contrôle (6) et la contre-électrode (7) pouvant être alimentées avec la tension du générateur de haute tension par l'intermédiaire d'un dispositif de commande et de mesure, et le courant qui circule pouvant être mesuré et déterminé par le dispositif de mesure.

10. Dispositif selon la revendication 9, **caractérisé en ce que** la broche de contrôle (6) est conformée en aiguille creuse pour alimenter le cylindre de seringue avec un gaz avant et/ou après le contrôle.

11. Dispositif selon la revendication 9 ou 10 , **caractérisé en ce que** la broche de contrôle (6) est pourvue d'un isolant (8) qui ne laisse que la pointe dégagée.

12. Dispositif selon les revendications 9 à 11 , **caractérisé en ce qu'**il est prévu de préférence quatre ou six postes de contrôle (1), chaque poste, pour le contrôle simultané dans chacun des postes de contrôle (1), comportant une alimentation en courant propre et un dispositif de mesure de courant.

13. Dispositif selon les revendications 9 à 12 , **caractérisé en ce que** la contre-électrode (7) est conformée en profilé en U.

14. Dispositif selon les revendications 9 à 12 , **caractérisé en ce que** la contre-électrode (7) est conformée en calotte sphérique entourant le capuchon de protection d'aiguille (2).

15. Dispositif selon les revendications 9 à 14 , **caractérisé en ce que** le générateur de haute tension est agencé pour produire une tension continue de l'ordre de 10 kV, de préférence de 12 à 14 kV.

16. Dispositif selon les revendications 9 à 15 , **caractérisé en ce qu'**un poste de décharge (9) pour éliminer la charge électrostatique des seringues (3) est disposé après le ou les postes de contrôle (1), lequel poste de décharge produit un nuage de décharge ionisé qui est traversé par les seringues (3).

17. Dispositif selon la revendication 16 , **caractérisé en ce que** le poste de décharge (9) est formé d'une tête d'ionisation (12) pourvue d'une ou de plusieurs pointes d'électrodes (10), les pointes d'électrodes étant alimentées avec une tension alternative d'environ 5 kV.

18. Dispositif selon la revendication 17 , **caractérisé en ce que** les pointes d'électrodes (10) sont disposées en cercle les unes par rapport aux autres.

19. Dispositif selon la revendication 17 , **caractérisé en ce que** la tête d'ionisation (12) est conformée en barre, de forme rectangulaire, carrée ou ovale.

20. Dispositif selon la revendication 17, 18 ou 19, **caractérisé en ce que** les pointes d'électrodes (10) sont disposées d'une manière adaptée à la forme du corps de seringue (3) et le cas échéant de l'unité support (13)qui porte le corps de seringue.

21. Dispositif selon une des revendications 17 à 20, **caractérisé en ce que** des ouvertures de soufflage (11) sont prévues qui sont alimentées par une arrivée de gaz commune.
